# EUROPEAN PATENT APPLICATION

(11) **EP 4 353 215 A1**
(43) Date of publication of application: **17.04.2024**
(21) Application number: 22841539.4
(22) Date of filing: 27.04.2022
(51) Int. Cl.: A61H 1/02, A61N 2/06, A63B 21/00, A63B 21/02, A63B 21/04, A63B 21/045, A63B 23/16

(54) **ANTI-STRESS AND/OR HAND REHABILITATION DEVICE**

(30) Priority: 14.07.2021 ES 202131472 U
(71) Applicant: Ruiz Artazo, Walter Alfredo, Rivadavia, San Juan, 5400 (AR)
(72) Inventor: Ruiz Artazo, Walter Alfredo, Rivadavia, San Juan, 5400 (AR)
(74) Representative: Espiell Gomez, Ignacio
(86) International application number: PCT/ES2022/070259
(87) International publication number: WO 2023/285710

(57) **Abstract**

The present invention relates to an anti-stress and/or hand rehabilitation device comprising two support bases (2) holding a rod (3) with rounded rotating parts (4) incorporating magnets (5) which are fixed or have a movable attachment (5a) which allows pressure to be exerted on the magnets. It optionally comprises, coupled in a fixed or detachable manner at each of the ends of the rod (3), a spring (7) or the like, at the distal end of which there is coupled a side gripping part (8) with four circular holes (8a) for inserting fingers therein.

## Description

### OBJECT OF THE INVENTION

The invention, as stated in the title of the present specification, relates to an anti-stress and/or hand rehabilitation device that contributes advantages and features to the function intended, which are described in detail further on.

The object of the present invention relates to a device the structure of which, envisaged for being placed on a table and comprising the inclusion of one or more bodies provided with magnets that rotate about a shaft when passing hands over same, is specifically designed as relaxing means for channeling tension and stress, for example in the office, as it helps to massage the nerve endings of the hands, serving as a magnetotherapy tool and which can likewise serve as means for exercising and rehabilitating the hand and adjacent areas, such as the wrist and forearm, and for preventing diseases caused by office work and/or similar activities by releasing the contraction of muscles in the hand and/or wrist.

### FIELD OF APPLICATION OF THE INVENTION

The field of application of the present invention is comprised in the sector of the industry dedicated to the manufacture of devices, implements, and tools for channeling stress and for performing hand exercises.

### BACKGROUND OF THE INVENTION

As is known, different types and models of implements and devices intended for exercising the hands to relieve stress and/or to strengthen and move hand muscles are available on the market. They are usually mechanically-operated apparatus:
- which are formed by an elastic body that is squeezed by hand, for example in the form of a ball, a sphere, an egg, or a net, or
- which include parts attached by springs for pressing with hand or with fingers, or
- which are in the form of a flexible bar formed by a spring with grips for pressing with both hands.

Relaxing or anti-stress devices or implements normally formed by mobile elements for moving, touching, and/or pressing, for example a cube in the form of a dice with buttons for pressing, or for example toys in the form of spheres, etc., are likewise known.

Moreover, the action that magnetism exerts on the body is known. In fact, magnetotherapy is a therapeutic treatment based on the application of magnetic fields commonly used by physiotherapists and rehabilitation centers.

In turn, magnetic therapy, magnetotherapy, or biomagnetism is an alternative medical practice involving the use of static magnetic fields produced by permanent magnets that has beneficial effects on health.

The objective of the present invention is therefore to provide the market with a new device which combines, in one and the same element:
- anti-stress means, for achieving user relaxation, particularly in the professional field, for users who work in offices for long hours and who almost always do so by using their hands to type on the computer and/or to hold the mouse, to enable relieving tensions through the channeling of nerve endings with the effect of magnets, and
- massaging and rehabilitation means, for activating and strengthening muscles in the hand as a whole, as well as in the fingers, and even the wrist and forearm.

Moreover, and as a reference to the current state of the art, although it has been mentioned that other devices are known, at least the applicant itself is unaware of the existence of any device which combines both things in a single device, or any other device which has technical and structural characteristics that are identical or similar to those specifically presented by the device claimed herein.

### DESCRIPTION OF THE INVENTION

The anti-stress and/or hand rehabilitation device proposed by the invention is configured as the ideal solution to the aforementioned objective, the characterizing details that make it possible and that distinguish it being conveniently included in the final claims that accompany this description.

Specifically, as indicated above, the invention proposes a device the structure of which with rotating elements provided with magnets is specifically designed as relaxing means for channeling tension and stress caused by the use of computers or other manual elements, for example in the office, as it helps to massage the nerve endings of the hands, serving at the same time as a magnetotherapy tool, and which furthermore, optionally, can also be used for hand exercise and rehabilitation, as well as for preventing muscle diseases of the hand and fingers, as well as the wrist or forearm.

To that end, and more specifically, the device essentially comprises two support bases between which there is held a metal rod on which there are inserted, with the possibility of rotating about same like a shaft, one or more rounded parts, preferably three parts in the form of sphere, in which magnets are in turn incorporated, radially distributed on the surface thereof.

Therefore, for a preferred use of the device, the user grasps with his/her index, middle, ring, and little fingers the bases of the article using both hands and with his/her thumbs rotates the spheres with magnets threaded on the central rod.

Furthermore, in the preferred embodiment, the device also comprises, coupled at the respective ends of the rod acting as a shaft for the rotating bodies with the magnets, a spring or a similar flexible filiform element, and at the distal end thereof a side gripping part, preferably provided with four hollow circles suitable for placing the (index, middle, ring, and little) fingers inserted therein.

Therefore, for a preferred use of the device, the user, after supporting the device on a table or a similar flat surface, should place his/her fingers in the circles of the two gripping parts at the ends, and he/she will be able to perform movements in any direction that the flexibility of the springs allows. This allows tensioning the muscles of the hand and expanding them. The user will also be able to perform lateral movements, up and down movements (which also allows the user to work the muscles of the wrist and some of the forearm), and finally the possibility of contracting more than one finger at a time.

Likewise, the user will also be able to perform movements with the palm of his/her hand on the rotating spheres with the magnets, causing a relaxing effect on the nerve endings of the palm.

It should be mentioned at this point that, although the magnets are preferably incorporated integrally on the surface of the rotating parts, optionally one, several, or all the magnets are incorporated by means of a moving, rotating, or flexible attachment, for example a strap, allowing the magnets to be pressed with the hand or fingers and/or to be rotated with respect to the spheres, and therefore offering in this manner a new muscle relaxing and/or exercising functionality.

Moreover, it should be noted that, in a preferred embodiment, the springs with the side gripping part with holes for the fingers are detachable elements so that they can be removed and used independently of the rest of the device, allowing the performance of pressure exercises against the palm of the hand with each of them, which can serve to activate the muscles of the hand and forearm, strengthening them so as to prevent injuries. In turn, being detachable allows the device, i.e., the rod with the rotating parts with magnets which is held between the support bases, to be used without side grips to perform magnetotherapy and relaxation exercises by gently passing the hand over them.

In terms of the support bases, which optionally are also detachable elements, allowing the device to take up less space for storage and transport, in a preferred embodiment, they are each formed by two articulated parts that allow expanding the lower support surface on which the incorporation of a non-slip material has in turn been preferably envisaged.

Finally, it should be noted that, the side gripping parts with holes for fingers are preferably made of silicone, plastic, rubber, or another similar soft material, to prevent them from causing damage, while the rest of the parts will preferably be metal parts although the possibility of them also being made of plastic material is not ruled out.

### DESCRIPTION OF THE DRAWINGS

To complete the description provided herein, and for the purpose of helping to make the features of the invention more readily understandable, this description is accompanied by a set of drawings constituting an integral part of the same, which by way of illustration and not limitation represents the following:
Figure 1 shows a schematic top plan view of an embodiment of the anti-stress and/or hand rehabilitation device object of the invention, in which the parts and elements which it comprises, as well as the arrangement thereof, can be seen;
Figure 2 shows a side elevational view of the example of the anti-stress and/or hand rehabilitation device of the invention shown in Figure 1;
Figure 3 shows a top perspective view of the example of the device according to the invention shown in the preceding figures, in this case including arrows indicative of the different movements of some of its portions;
Figures 4, 5, and 6 show another example of the device according to the invention in the same views as Figures 1, 2, and 3, in this case an example in which it does not comprise the springs or the side gripping parts;
Figure 7 shows a perspective view of another example of the device of the invention, in this case an example with double support bases, mobile magnets, and removable side gripping parts;
Figures 8-A and 8-B show respective perspective views of one of the rotating parts of the device, according to the example of Figure 7, with mobile magnets depicted respectively in a raised position and in a retracted position; and
Figure 9 shows a perspective view of one of the removable grips of the example of the device shown in Figure 7, depicted independent of the rest of the device.

### PREFERRED EMBODIMENT OF THE INVENTION

In view of the aforementioned figures, and in accordance with the adopted numbering, one may observe therein several non-limiting exemplary embodiments of the anti-stress and/or hand rehabilitation device of the invention, comprising that which is described in detail below.

In that sense, as seen in said figures, the device (1) of the invention basically comprises two support bases (2), suitable for holding the device (1) raised a certain distance over a flat horizontal surface (s), between which there is held, also in a horizontal position, a rod (3) on which there are inserted, with a shaft-like rotational movement, one or more rounded rotating parts (4) in which magnets (5) are in turn incorporated, fixed on the surface thereof, such that the user, when passing his/her hand or fingers over said parts (4) causing them to rotate on the rod (3), receives the magnetic effect of the magnets (5).

Preferably, the magnets (5) are formed by circular parts projecting above the round surface of the rotating parts (4) on which they are fixed, massaging the user's hands.

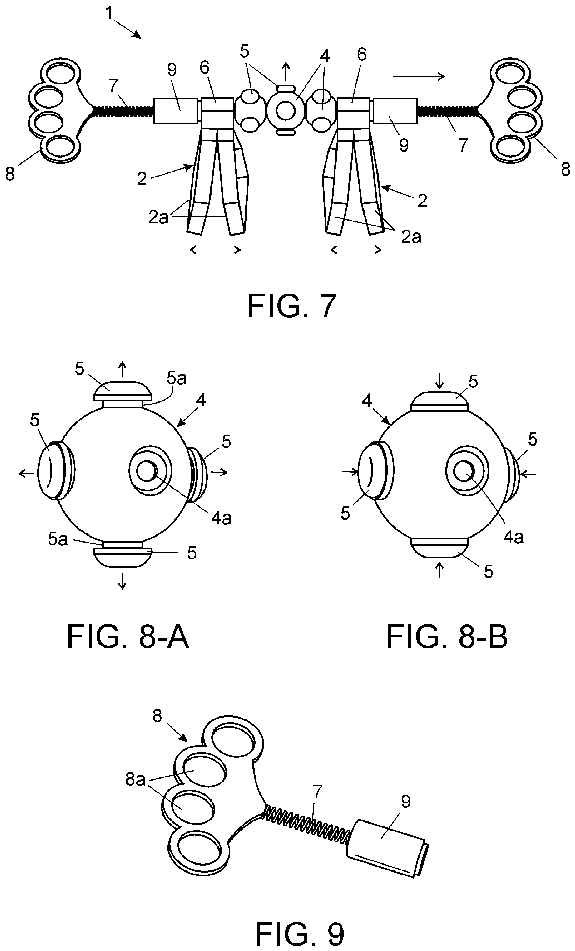

In one embodiment, the magnets (5) are attached integrally to the surface of the rotating parts (4) in a fixed and immobile manner. However, in an alternative embodiment option, one, several, or all the magnets (5) are incorporated on the surface of the rotating parts (4) by means of a movable attachment (5a), for example a spring, a strap, or a similar system, which allows pressure to be exerted on the magnets and tends to return them to their initial position at all times, as seen in Figures 8-A and 8-B.

Preferably, the rotating parts (4) are spherical and have a through opening (4a) for insertion on the rod (3).

Preferably, the rod (3) incorporates three rotating parts (4).

Preferably, the support bases (2) are detachable.

Preferably, the support bases (2) are fixed by means of snap-fitting, in cylindrical bushings (6) in which the ends of the shaft-like rod (3) are fixed.

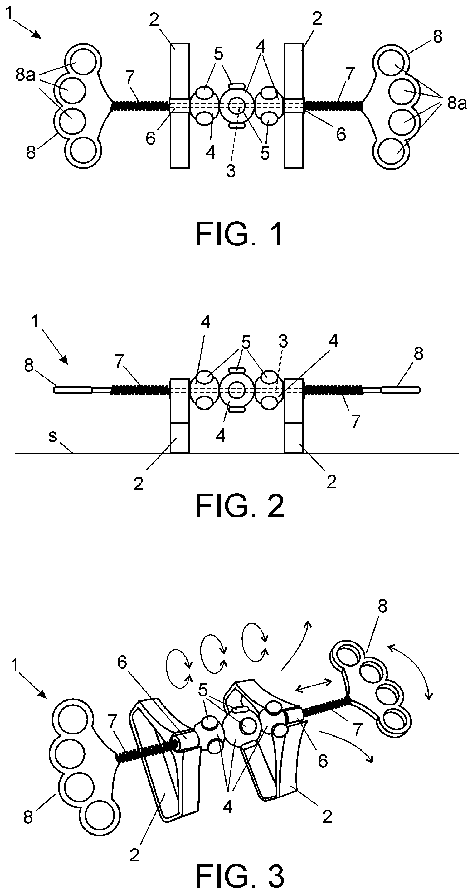

In a preferred embodiment, the detachable or non-detachable bases (2) are each formed by a single part, as shown by the example of Figures 1 to 6.

Furthermore, in an alternative embodiment, such as that shown in Figure 7, each of the two bases (2) of the device is formed by two parts (2a) articulated to one another at their upper portion, where a bushing (6) links them to the rod (3), such that they can be further separated at the lower portion thereof, where they define the support of the assembly, so as to provide a larger holding surface.

Preferably, the bases (2), whether formed both by one part or by two parts (2a), incorporate on the lower face thereof a layer of non-slip material, to prevent the device from moving when using same.

In a preferred embodiment, the device (1) also comprises, coupled at each of the respective ends of the rod (3) acting as a shaft for the rotating parts (4), a spring (7) or a similar flexible filiform element, at the distal end of which there is coupled a side gripping part (8), intended for being held with the hands, for which it is provided with openings, specifically with four circular holes (8a), suitable for inserting (index, middle, ring, and little) fingers therein in order to apply movements in any direction so as to overcome the force of the springs (7) and thereby perform rehabilitation exercises.

As shown by the arrows of Figure 3, said movements can be both compressive, towards the central part of the device, and extensive in the opposite direction, such as upwards and downwards or towards one side or the other. Logically, the amplitude of such movements will depend on the length of the springs (7) and on the degree of flexibility thereof, both of which may vary depending on manufacturer's preferences in each case.

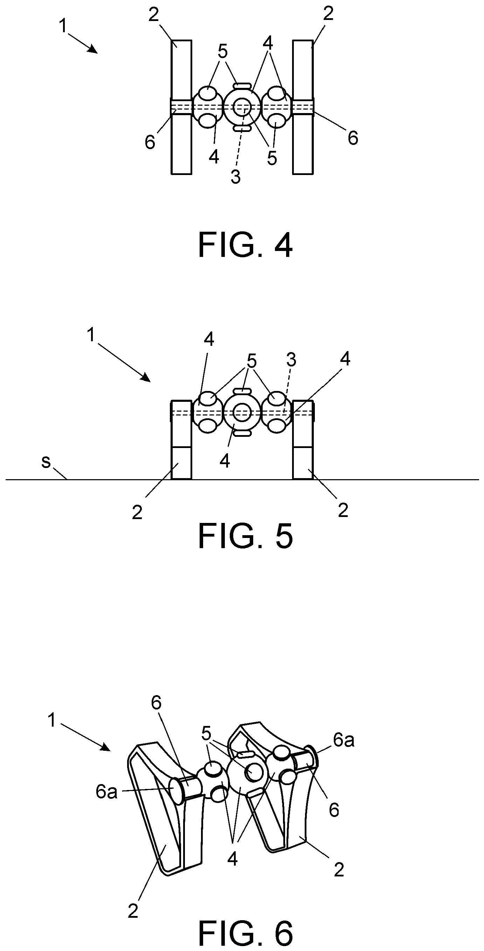

In the case where the device (1) does not comprise the springs (7) or the gripping parts (8), preferably as shown in Figures 4, 5, and 6, the device incorporates two additional covers (6a) for covering the external openings of the bushings (6) of the bases (2) where said springs (7) would be arranged, with the central device being suitable to be used only for massage.

Moreover, as shown by the example of Figure 7, in an embodiment option, the springs (7) with the gripping parts (8) are detachable, allowing the independent use thereof (Figure 9) as well as the use of the rest of the device (1) without same.

To that end, the end of the spring (7) attached to the rod (3) preferably has a stop (9) which, in addition to being used for the removable coupling thereof to said rod (3), preferably through a bushing (6) in which the bases (2) are fixed, for example by means of threading or by means of pressure, in turn also serves for using the gripping part (8) to perform exercises with the hand, specifically by inserting fingers in the holes (8a) and supporting said stop (9) on the palm of the hand for pressing by closing the hand and for overcoming the force of the spring (7). In the absence of said stop (9), if the end of the spring (7) is supported directly, it could cause damage to the user as it is sharp and made of metal.

Moreover, preferably both the gripping part (8) and the described stop (9) at the end of the spring (7) are made of silicone, plastic, rubber, or another soft material, to prevent them from being able to cause damage.

Moreover, the dimensions of the device are likewise variable, although, in the preferred embodiment, the distance between the gripping parts (8) at the ends of the device and the rotating parts (4) of the rod (3) acting as a shaft is such that an adult user, upon introducing his/her four other fingers in the circular holes (8a) thereof with each hand, can touch the surface with magnets (5) of said rotating parts (4) with his/her thumbs.

Having sufficiently described the nature of the present invention, as well as the ways in which it may be implemented, it is not considered necessary to elaborate on the explanation thereof in order for a person skilled in the art to understand the scope of the invention and the advantages derived therefrom.

## Claims

1. An anti-stress and/or hand rehabilitation device, **characterized by** comprising two support bases (2) between which there is held a rod (3) on which there are inserted, with a shaft-like rotational movement, one or more rounded rotating parts (4) in which magnets (5) are in turn incorporated, fixed on the surface thereof.

2. The anti-stress and/or hand rehabilitation device according to claim 1, **characterized in that** the magnets (5) are made up of circular parts projecting above the round surface of the rotating parts (4) on which they are fixed.

3. The anti-stress and/or hand rehabilitation device according to claim 1 or 2, **characterized in that** the magnets (5) are attached integrally to the surface of the rotating parts (4) in a fixed and immobile manner.

4. The anti-stress and/or hand rehabilitation device according to claim 1 or 2, **characterized in that** one, several, or all the magnets (5) are incorporated on the surface of the rotating parts (4) by means of a movable attachment (5a) which allows pressure to be exerted on the magnets and tends to return them to their initial position at all times.

5. The anti-stress and/or hand rehabilitation device according to any of the preceding claims, **characterized in that** the rotating parts (4) are spherical.

6. The anti-stress and/or hand rehabilitation device according to any of the preceding claims, **characterized in that** the rod (3) incorporates three rotating parts (4).

7. The anti-stress and/or hand rehabilitation device according to any of the preceding claims, **characterized in that** the support bases (2) are detachable.

8. The anti-stress and/or hand rehabilitation device according to claim 7, **characterized in that** the support bases (2) are fixed, by means of snap-fitting, in cylindrical bushings (6) in which the ends of the rod (3) are fixed.

9. The anti-stress and/or hand rehabilitation device according to any of the preceding claims, **characterized in that** the bases (2) are each formed by a single part.

10. The anti-stress and/or hand rehabilitation device according to any of claims 1 to 8, **characterized in that** the bases (2) are each formed by two parts (2a) articulated to one another at their upper portion such that they can be separated at their lower portion to provide a larger holding surface.

11. The anti-stress and/or hand rehabilitation device according to any of the preceding claims, **characterized in that** it comprises, coupled at each of the respective ends of the rod (3) acting as a shaft for the rotating parts (4), a spring (7) or a similar flexible filiform element, at the distal end of which there is coupled a side gripping part (8) provided with openings consisting of four circular holes (8a) suitable for inserting fingers therein.

12. The anti-stress and/or hand rehabilitation device according to claim 11, **characterized in that** the springs (7) with the gripping parts (8) are detachable, allowing the independent use thereof, as well as the use of the rest of the device (1) without same.

13. The anti-stress and/or hand rehabilitation device according to claim 12, **characterized in that** the end of the spring (7) attached to the rod (3) has a stop (9) that is used for the removable coupling thereof and also for supporting said stop (9) on the palm of the hand.

14. The anti-stress and/or hand rehabilitation device according to claim 13, **characterized in that** both the gripping part (8) and the stop (9) at the end of the spring (7) are made of silicone, plastic, rubber, or another soft material.

15. The anti-stress and/or hand rehabilitation device according to any of claims 11 to 14, **characterized in that** the distance between the gripping parts (8) and the rotating parts (4) of the rod (3) is such that an adult user, upon introducing his/her four other fingers in the circular holes (8a) thereof with each hand, can touch the surface with magnets (5) of said rotating parts (4) with his/her thumbs.
